# EUROPEAN PATENT APPLICATION

(11) **EP 4 759 322 A1**
(43) Date of publication of application: **17.06.2026**
(21) Application number: 24218895.1
(22) Date of filing: 10.12.2024
(51) Int. Cl.: A61K 36/752, A23L 33/105, A61K 9/00, A61K 31/7048, A61P 1/00, A61P 37/00

(54) **COMPOSITIONS FOR TREATING INTESTINAL BOWEL DISEASES**

(71) Applicant: ITALBIOTEC SRL SOCIETA' BENEFIT, 20126 Milan (IT)
(72) Inventor: TROMBETTA, Domenico, Reggio Calabria (IT); SMERIGLIO, Antonella, Messina (IT)
(74) Representative: Zaccaro, Elisabetta

(57) **Abstract**

The present invention concerns the field of nutraceutical medicine suitable for providing effective anti-inflammatory benefits for patients affected by inflammatory bowel diseases.

Specifically, the invention relates to nutraceutical and/or pharmaceutical formulation and uses thereof for treating intestinal inflammation.

## Description

### FIELD OF THE INVENTION

The present invention concerns the field of nutraceutical medicine suitable for providing effective anti-inflammatory benefits for patients affected by inflammatory bowel diseases.

Specifically, the invention relates to nutraceutical and/or pharmaceutical formulation and uses thereof for treating intestinal inflammation.

### STATE OF THE ART

Flavanones represent the most widespread subclass of flavonoids in the *Citrus* genus, and their antioxidant and anti-inflammatory properties are undoubted and universally recognized. These promising properties make flavanones important candidates in the treatment of various chronic inflammatory disorders such as cardiovascular and metabolic diseases.

In the last decade, flavanones have been the subject of in-depth studies for the treatment of intestinal bowel diseases (IBD) and Irritable Bowel Syndrome (IBS) due to the increase in their incidence globally, in relation to the emergence of new therapeutic strategies that limit the range of inter-individual variability in the therapeutic response, and to counteract the incidence and severity of the side effects of conventional drugs.

Indeed, several epidemiological studies show that there is a direct correlation between a flavanones-rich diet and decrease in IBD incidence. This is probably due to their activity as inhibitors of various proteins and inflammatory pathways, their antioxidant and free-radical scavenging effects, and their ability to influence the composition of the intestinal microbiota. These health properties have also been demonstrated *in vivo* by several studies on mouse models of IBD, where the administration of extracts and juices of *Citrus* fruits, or their most representative flavanones, confirm their ability to counteract oxidative stress and inflammation.

Although a rather vast body of literature on the subject is available, to date, only one study has evaluated and compared the antioxidant and anti-inflammatory effects of the most representative flavanones of the *Citrus* genus (Denaro, M.; Smeriglio, A.; Trombetta, D. Antioxidant and anti-inflammatory activity of citrus flavanones mix and its stability after in vitro simulated digestion. Antioxidants 2021, 10, 140). Indeed, except for naringenin and hesperidin, the other flavanones remain poorly investigated.

Furthermore, the information regarding the bio accessibility of this class of molecules remains rather lacking, and knowledge about their intestinal local anti-inflammatory action, systemic absorption and the mechanisms involved in the transport of these molecules through the gastrointestinal tract, as well as their fate following digestion, would be desirable.

Recently, in Denaro et al. (Denaro, M.; Smeriglio, A.; Trombetta, D. Antioxidant and anti-inflammatory activity of citrus flavanones mix and its stability after in vitro simulated digestion. Antioxidants 2021, 10, 140) an antioxidant and anti-inflammatory screening was carried out on the most representative flavanones of the *Citrus* genus, highlighting which molecules were the most promising. Furthermore, the same tests were also carried out on a mix of the most powerful flavanones (FM), to evaluate the potential combination effect, which has been demonstrated experimentally. FM was also subjected to *in vitro* simulated digestion (DFM) to evaluate the potential intestinal bio accessibility of these molecules, demonstrating how the investigated flavanones can reach the intestinal epithelium unchanged, where they can exert strong anti-inflammatory properties, as demonstrated on Caco-2 monolayer model, in which they significantly decreased the release of various inflammatory mediators such as interleukin (IL)-6, IL-8, and nitric oxide (NO). To investigate and postulate a feasible anti-inflammatory mechanism of action of these molecules and to investigate the ability of these five flavanones and DFM to counteract oxidative stress in the same Caco-2 cell-based model mentioned above, a subsequent study was carried out (Smeriglio, A.; Iraci, N.; Denaro, M.; Mandalari, G.; Giofrè, S.V.; Trombetta, D. Synergistic Combination of Citrus Flavanones as Strong Antioxidant and COX-Inhibitor Agent. Antioxidants 2023, 12, 972). This study specifically evaluated *in vitro* the inhibitory activity of each flavanone (neoeriocitrin, eriocitrin, hesperetin, hesperidin, and neohesperidin) and DFM on the two human COX isoforms (COX-1 and COX-2), as well as the downstream activity by recording the release of PGE2 by Caco-2 cells after treatment with arachidonic acid.

Furthermore, molecular modelling studies were carried out to highlight any interactions at the active, or other binding sites, of the enzymes' isoforms. Finally, the ability of flavanones and DFM to counteract cellular oxidative stress was investigated by monitoring the protein carbonyl content, thiobarbituric acid-reactive substances (TBARS), reactive oxygen species (ROS), and reduced glutathione/oxidized glutathione ratio (GSH/GSSG), evaluating also experimentally the potential synergistic effect of DGM.

This last study demonstrated how all the five flavanones exhibit strong antioxidant activity by reducing the ROS release, the formation of carbonylated proteins and lipid peroxides, and the oxidation of GSH to GSSG in Caco-2 cells. They were also able to exert strong anti-inflammatory activity by inhibiting COX enzymes, with a selectivity towards COX-2, as also demonstrated by molecular modelling studies, and consequently, the release of prostaglandins with an efficacy like the reference COX-2 selective drug nimesulide in Caco-2 cells.

Considering the results of previous studies highlighting the antioxidant and anti-inflammatory properties of *Citrus* flavanones (eriocitrin, neoeriocitrin, hesperidin, neohesperidin, and hesperetin), and their synergistic biological effects after *in vitro* simulated gastro-intestinal digestion, there is a challenge in developing nutraceutical formulations for inflammatory bowel diseases (IBD) and for Irritable Bowel Syndrome (IBS) for human use. This is due to regulatory restrictions in many jurisdictions, including Italy, on the use of certain synthetic flavanones (eriocitrin, neoeriocitrin, and neohesperidin) in food supplements.

The problem to be solved is to design a novel nutraceutical and pharmaceutical formulation that leverages the synergistic potential of permitted flavanones to provide effective anti-inflammatory benefits for IBD and IBS patients while adhering to regulatory constraints.

### SUMMARY OF THE INVENTION

Therefore, the solution proposed herein for the aforementioned object, is a composition comprising flavanones having antioxidant and anti-inflammatory properties, and in particular a nutraceutical composition consisting of:
- Hesperidin;
- Eriocitrin;
- Neoeriocitrin
- Neohesperidin; and
- at least one physiologically acceptable excipient,
wherein said Eriocitrin, Neoeriocitrin and Neohesperidin are from a *Citrus* extract. In a further aspect, the invention describes a pharmaceutical composition consisting of:
- Hesperidin;
- Eriocitrin;
- Neoeriocitrin
- Neohesperidin; and
- at least one physiologically acceptable excipient,
wherein said Eriocitrin, Neoeriocitrin and Neohesperidin are from a *citrus* extract. In a still further aspect, the invention relates to a pharmaceutical composition, as herein described, for use as a medicament.

It is also object of the invention the use of a pharmaceutical composition, as herein disclosed, in the prevention and/or treatment of Intestinal Bowel Disease (IBD) and Irritable Bowel Syndrome (IBS).

According to another aspect, the described invention provides the use of the nutraceutical composition as a food supplement.

The present invention shed light not only on some crucial aspects and mechanisms underlying the antioxidant and anti-inflammatory properties of the nutraceutical and pharmaceutical formulation, but also on the potential for its clinical use in the prevention and treatment of inflammatory bowel diseases.

In particular, the compositions of the invention, with its peculiar characteristics, are able to counteract in a dose-dependent manner the onset and exacerbation of ulcerative colitis, with the highest dose showing, at the same dosage (100 mg/Kg/day), comparable and sometimes even more promising results than the reference drug sulfasalazine.

This suggests, without being bound to any theory, a rational use of these formulations for preventive purposes, as well as in co-treatment with conventional drugs to reduce their dosage and consequently also their side effects.

### BRIEF DESCRIPTION OF THE DRAWINGS

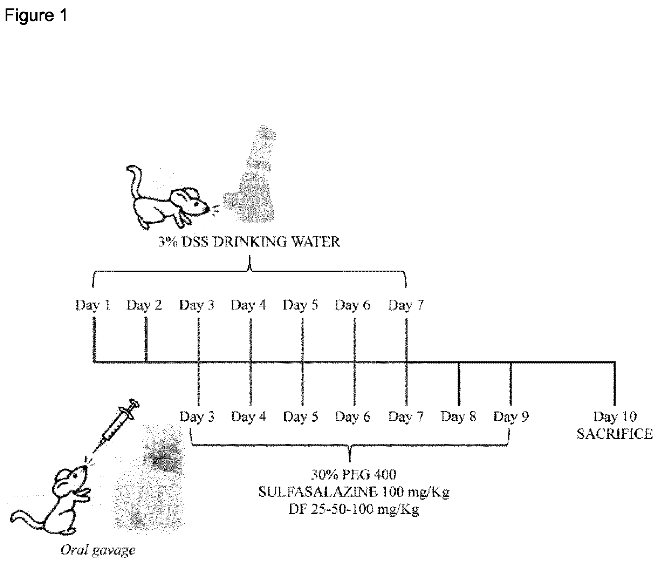

The characteristics and advantages of the present invention will be apparent from the detailed description reported below, from the Examples given for illustrative and non-limiting purposes, and from the annexed Figures 1-11, wherein:
Figure 1: experimental design of the *in vivo* study.

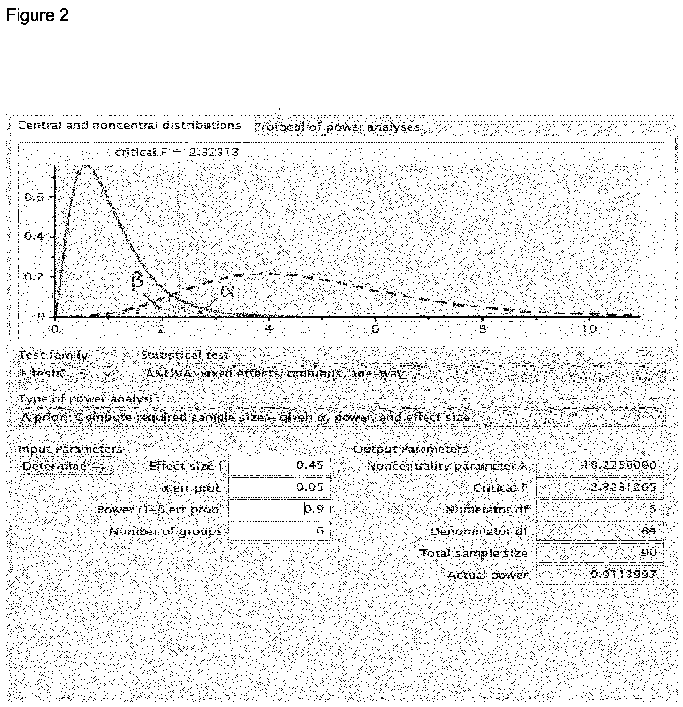

Figure 2: result of the calculation of the total sample size by G*Power software 3.1.9.4.

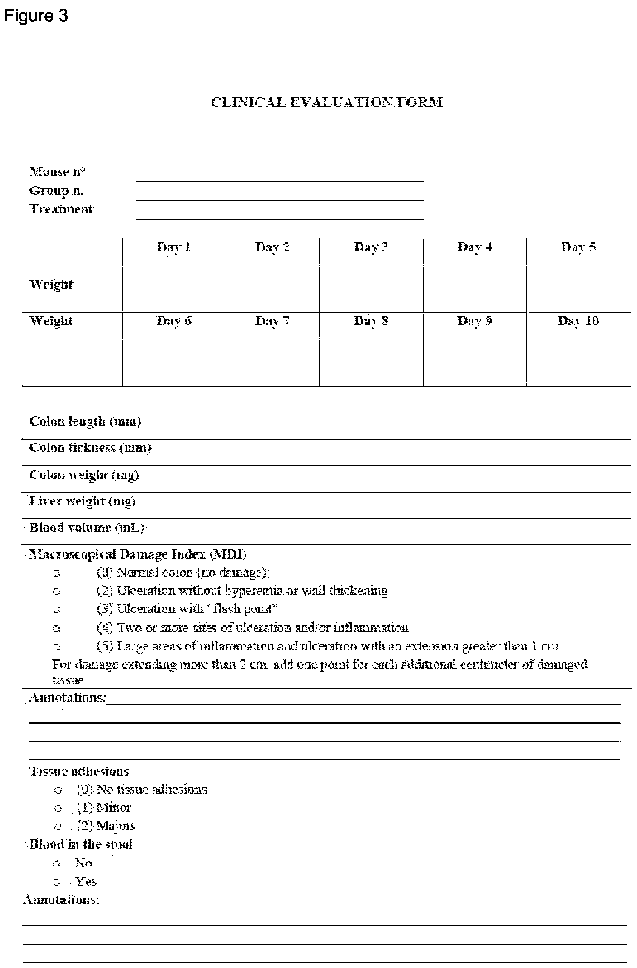

Figure 3: clinical evaluation form.

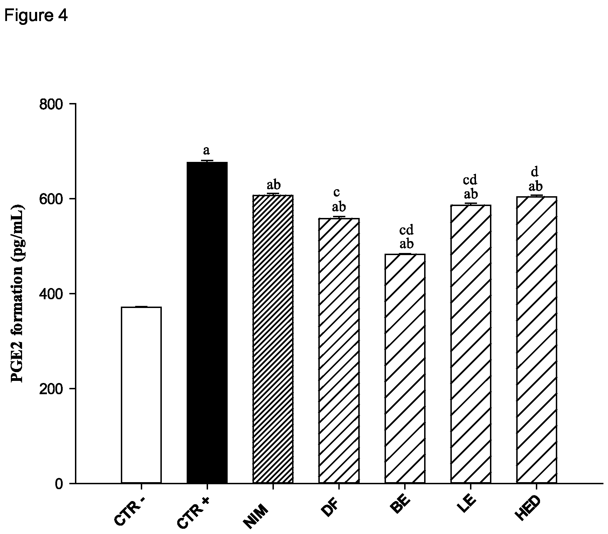

Figure 4: prostaglandin (PGE2) release upon exposure of Caco-2 monolayers to 10 mM arachidonic acid (AA) after treatment with 25 ng/mL IL-1β. CTR-, negative control treated only with 10 mM AA; CTR+, positive control treated both with 25 ng/mL IL-1β and 10 mM AA; NIM, 10 µM nimesulide, used as reference standard; DF, 10 µM digested formulation (7:1:2 as µM for HED, LE and BE); BE, Bergamot extract (10 µM neoeriocitrin and 10 µM neohesperidin); LE, 10 µM eriocitrin; HED, 10 µM hesperidin. ^{a}*p*<0.001 *vs*. CTR-; ^{b}*p*<0.001 vs. CTR+; ^{c}*p*<0.05 vs. NIM; ^{d}*p*<0.05 *vs*. DF.

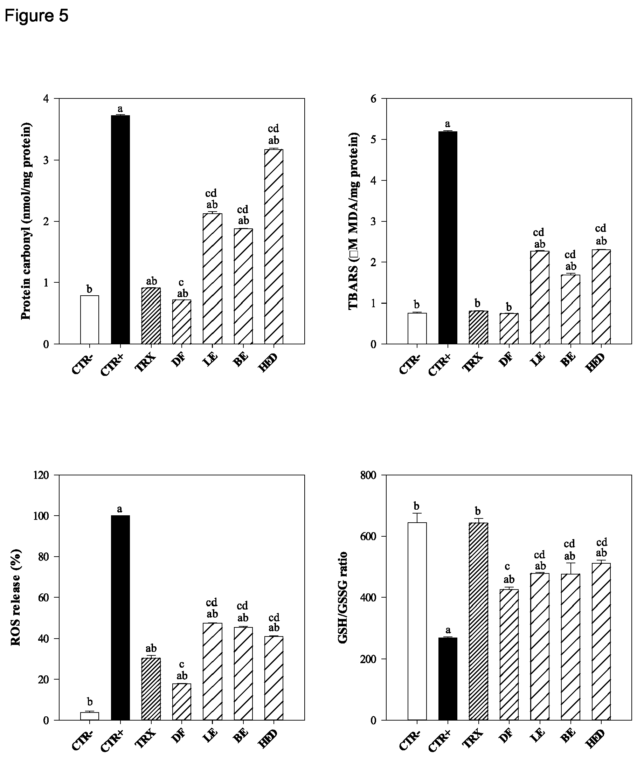

Figure 5: the exposure to 25 ng/mL IL-1β causes pro-oxidant response in Caco-2 monolayers. CTR-, negative control treated only with culture medium; CTR+, positive control treated with 25 ng/mL IL-1β; TRX, 10 µM trolox, used as reference standard; DF, 10 µM digested formulation (7:1:2 as µM for HED, LE and BE); BE, Bergamot Extract (10 µM neoeriocitrin and 10 µM neohesperidin); LE, Lemon Extract (10 µM eriocitrin); HED, 10 µM hesperidin. ^{a}*p*<0.001 *vs*. CTR-; ^{b}*p*<0.001 *vs*. CTR+; ^{c}p<0.05 *vs*. TRX; ^{d}p<0.001 *vs*. DF, except for GSH/GSSG p<0.05.

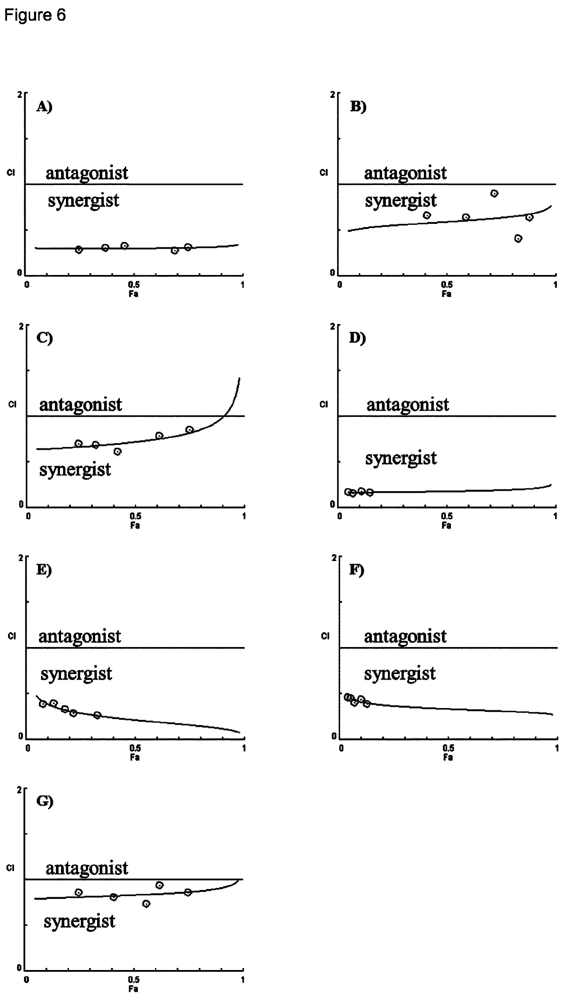

Figure 6: Screening of the synergistic effect of DF (2.5-20 µM) at constant ratio of 7:1:2 for HED (0.875-14 µM), LE (0.125-2 µM) and BE (0.25-4 µM). The simulated lines were generated from CompuSyn, by plotting Cl versus Fa values. A) COX-1, B) COX-2, C) GSH/GSSG, D) Protein carbonyl, E) ROS, F) TBARS and G) PGE2.

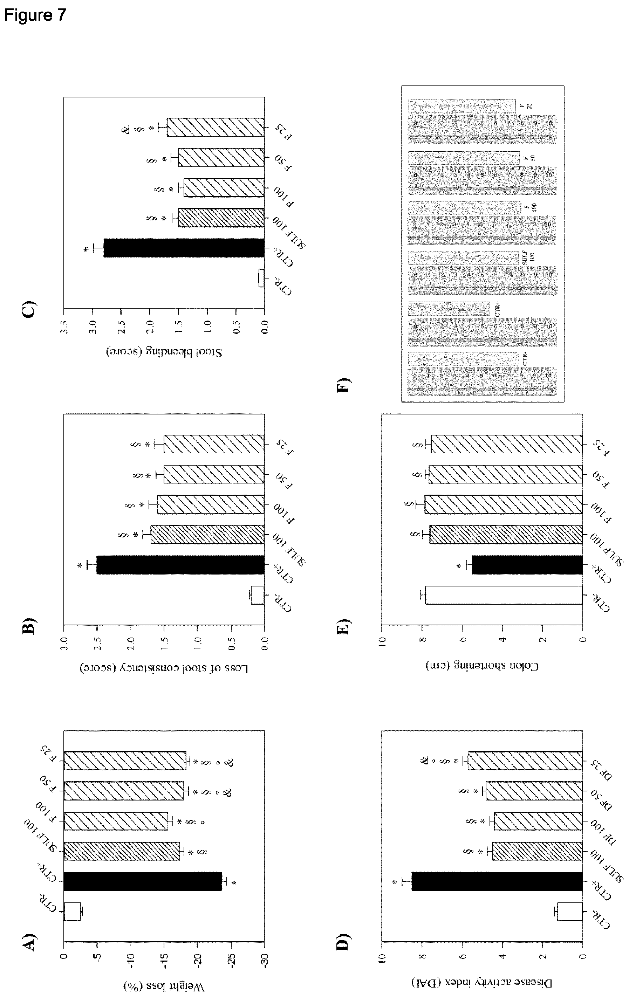

Figure 7: effects of the formulation (F) on macroscopic damage in mice with 3% DSS-induced UC. The severity of colitis was determined by the DAI score (D), which combines the scores obtained from body weight (A), stool consistency (B), and faecal bleeding (C) changes. These parameters were calculated as reported in Table 2. After sacrifice, the colon length (cm) between the colon-caecal junction and the rectum was recorded (E and F). **p* < 0.05 vs CTR- (vehicle group); ^{§}*p* <0.05 vs CTR+ (3% DSS-treated group); °*p* < 0.05 vs SULF 100 (sulfasalazine 100 mg/kg/day treated group); ^{&}*p* <0.05 vs F 100 (F 100 mg/kg/day treated group).

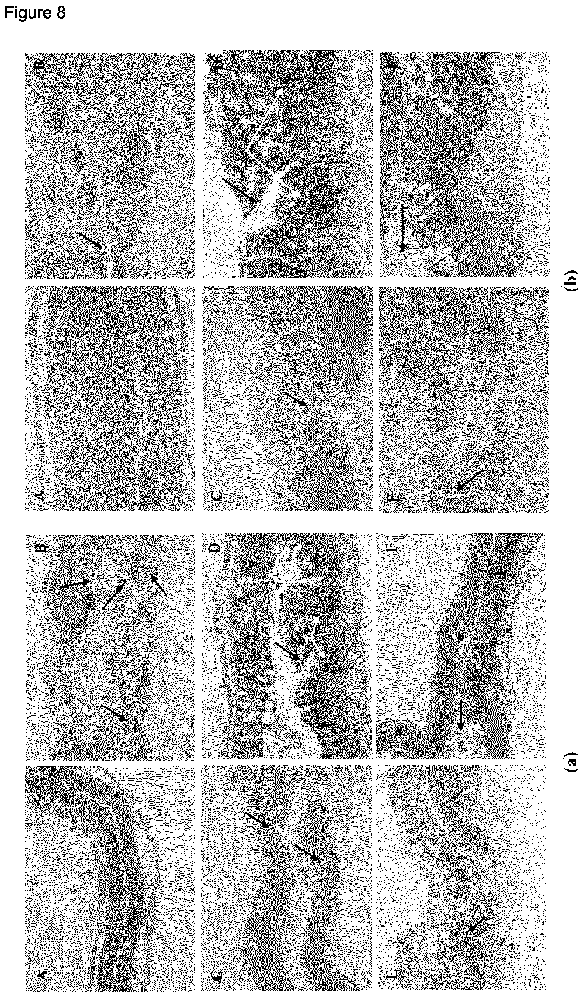

Figure 8: representative pictures of Hematoxylin and Eosin staining of colon tissues of each group at 4× (panel a) and 10× (panel b) magnification. A, CTR- (vehicle group); B, CTR+ (3% DSS); C, SULF 100 mg/Kg/day; D, F 100 mg/Kg/day; E, F 50 mg/Kg/day; F, F 25 mg/Kg/day.

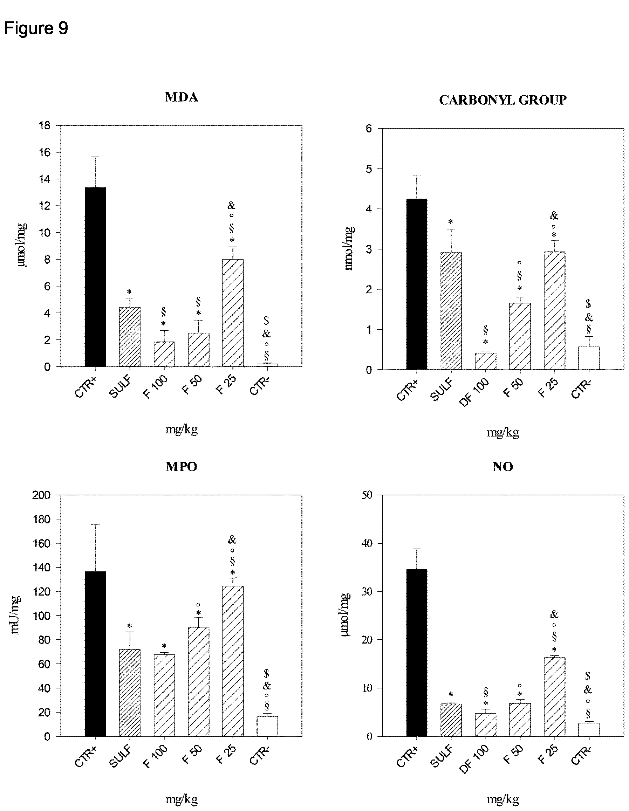

Figure 9: determination of pro-oxidant markers by colorimetric assays. The results, which represent the mean ± standard deviation of three independent experiments in triplicate (*n*=3), were expressed as pg/mg of protein. CTR+, 3% DSS-treated group; SULF, Sulfasalazine 100 mg/Kg; F, formulation 25, 50 and 100 mg/Kg; CTR, vehicle group. **p* < 0.05 *vs* CTR+; ^{§}p < 0.05 *vs* SULF 100 mg/kg; °*p* < 0.05 *vs* F 100 mg/kg; ^{&} *p* < 0.05 *vs* F 50 mg/kg; ^{$}*p* < 0.05 *vs* F 25 mg/kg.

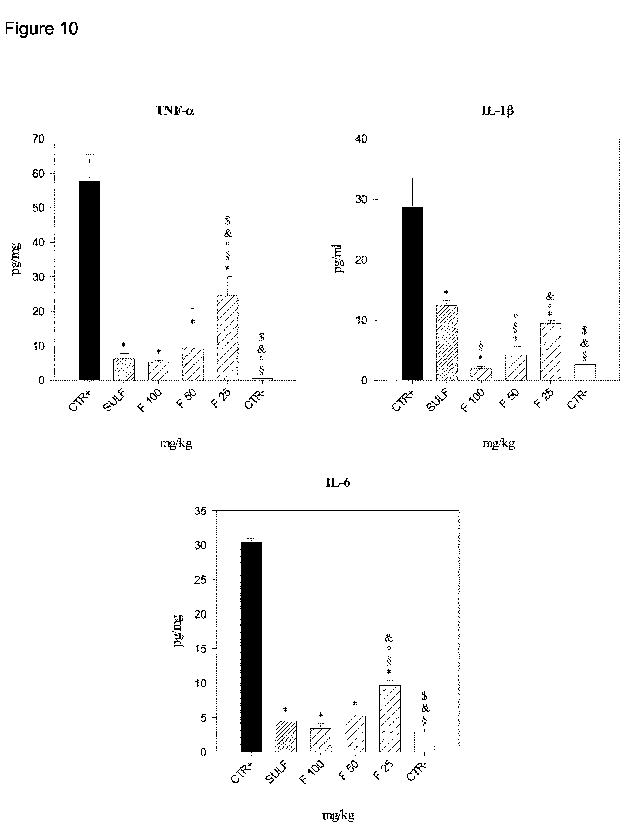

Figure 10: determination of pro-inflammatory markers by enzyme-linked immunosorbent assay (ELISA) in colon samples. The results, which represent the mean ± standard deviation of three independent experiments in triplicate (*n*=3), were expressed as pg/mg of protein. CTR+, 3% DSS-treated group; SULF, Sulfasalazine 100 mg/Kg; F, formulation 25, 50 and 100 mg/Kg; CTR, vehicle group. **p* < 0.05 vs CTR+; ^{§}*p* < 0.05 *vs* SULF 100 mg/kg; °*p* < 0.05 *vs* F 100 mg/kg; ^{&} *p* < 0.05 *vs* F 50 mg/kg; ^{$}*p* < 0.05 *vs* F 25 mg/kg.

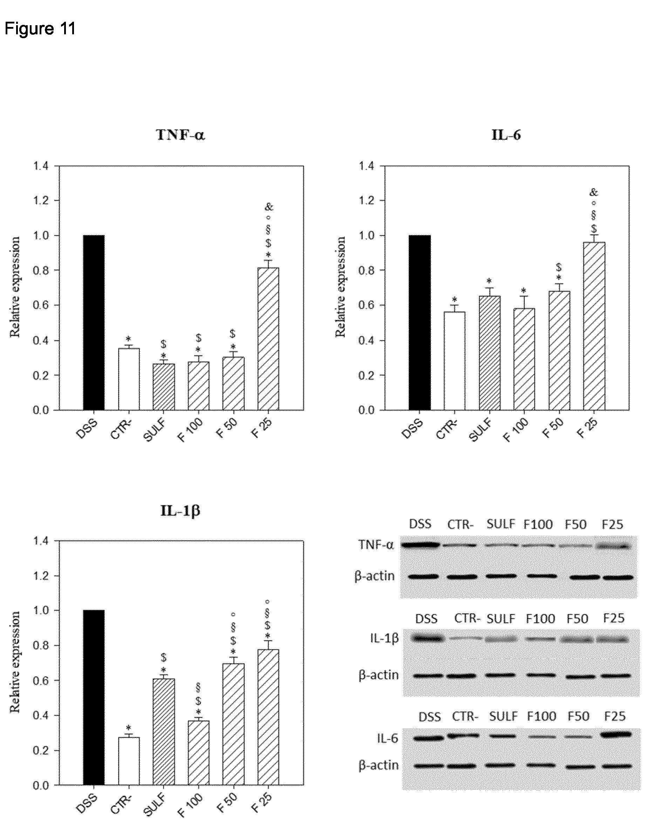

Figure 11: determination of pro-inflammatory markers proteins expression by wester blotting analyses. The results, which represent the mean ± standard deviation of three independent experiments in triplicate (*n*=3), were expressed as relative protein expression. DSS, 3% DSS-treated group; CTR, vehicle group; SULF, Sulfasalazine 100 mg/Kg; F, formulation 25, 50 and 100 mg/Kg. **p* < 0.05 *vs* DSS; ^{$}*p* < 0.05 *vs* CTR-; ^{§}*p* < 0.05 *vs* SULF 100 mg/kg; °*p* < 0.05 *vs* F 100 mg/kg; ^{&}*p* < 0.05 *vs* F 50 mg/kg.

### DETAILED DESCRIPTION OF THE INVENTION

The present invention concerns a nutraceutical composition consisting of:
- Hesperidin;
- Eriocitrin;
- Neoeriocitrin
- Neohesperidin; and
- at least one physiologically acceptable excipient,
wherein said Eriocitrin, Neoeriocitrin and Neohesperidin are from a *Citrus* extract. In a further aspect, the invention describes a pharmaceutical composition consisting of:
- Hesperidin;
- Eriocitrin;
- Neoeriocitrin
- Neohesperidin; and
- at least one physiologically acceptable excipient,
wherein said Eriocitrin, Neoeriocitrin and Neohesperidin are from a *Citrus* extract. Hesperidin is a flavanone glycoside (Hesperetin-7-rutinoside) found in *Citrus* fruits, and has the following structure: 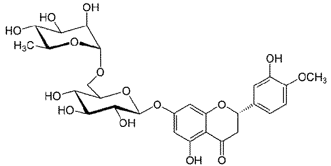 (2S)-5-hydroxy-2-(3-hydroxy-4-methoxyphenyl)-7-[(2S, 3R, 4S, 5S, 6R)-3,4,5-trihydroxy-6-[[(2R,3R,4R,5R,6S)-3,4,5-trihydroxy-6-methyloxan-2-yl]oxymethyl]oxan-2-yl]oxy-2,3-dihydrochromen-4-one.

Eriocitrin (Eriodictyol 7-O-rutinoside) is commonly found in lemons and other *Citrus* fruits and has this formula: 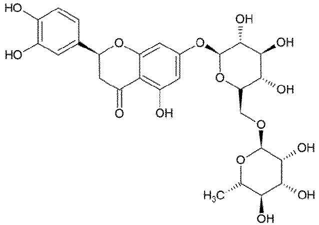 (2S)-2-(3,4-dihydroxyphenyl)-5-hydroxy-4-oxo-3,4-dihydro-2H-chromen-7-yl 6-O-(6-deoxy-alpha-L-mannopyranosyl)-beta-D-glucopyranoside.

Neoeriocitrin (Eriodictyol 7-O-neohesperidoside), that is a glycoside of the flavanone eriodictyol has the following structure: 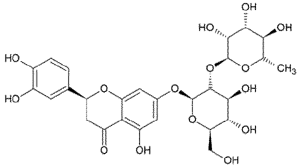 (2S)-7-[(2S,3R,4S,5S,6R)-4,5-dihydroxy-6-(hydroxymethyl)-3-[(2S,3R,4R,5R,6S)-3,4,5-trihydroxy-6-methyloxan-2-yl]oxyoxan-2-yl]oxy-2-(3,4-dihydroxyphenyl)-5-hydroxy-2,3-dihydrochromen-4-one.

Neohesperidin (Hesperetin-7-neohesperidoside) is the 7-O-neohesperidose derivative of the hesperetin, which in turn is the 4'-methoxy derivative of eriodictyol It has the following structure: 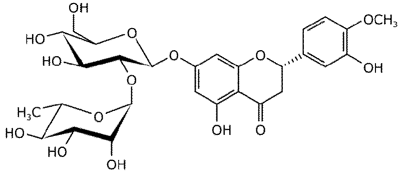 (S)-7-(((2-O-6-Deoxy-alpha-L-mannopyranosyl)-beta-D-glucopyranosyl)oxy)-2,3-dihydro-5-hydroxy-2-(3-hydroxy-4-methoxyphenyl)-4H-1-benzopyran-4-one.

In a preferred embodiment, in the nutraceutical composition or in the pharmaceutical composition of the invention, *Citrus* extract is selected from the group consisting of lemon extract or bergamot extract.

In a still preferred embodiment, in the nutraceutical or in the pharmaceutical composition the Eriocitrin is from lemon extract and the Neoeriocitrin and the Neohesperidin are from bergamot extract.

In order to provide effective anti-inflammatory benefits for IBD patients while adhering to regulatory constraints, synthetic eriocitrin, neoeriocitrin and neohesperidin flavanone molecules were replaced with two dry *Citrus* extracts, *Citrus aurantium* L. *var. bergamia* (Neoeriocitrin and Neohesperidin) and *Citrus* × *limon* (L.) Osbeck (Eriocitrin).

In a further preferred embodiment, in the nutraceutical composition according to the invention or the pharmaceutical composition herein described
- Hesperidin is in the range from 50 mg to 300 mg;
- Eriocitrin is in the range from 10 mg to 60 mg; and
- Neoeriocitrin and Neohesperidin are in the range from 20 mg to 100 mg. Preferably,
- Hesperidin is in the amount of 112 mg;
- Eriocitrin is in the amount of 22 mg; and
- Neoeriocitrin and Neohesperidin is in the total amount of 40 mg.

It was surprisingly found that the most promising ratios among Hesperidin, Lemon extract containing Eriocitrin and Bergamot extract containing Neoeriocitrin and Neohesperidin is 7:1:2.

In a still further preferred embodiment, the physiologically acceptable excipient of the compositions is selected from the group consisting of an auxiliary agent, a filler, an amino acid, a protein, a fatty acid, a carbohydrate, a vitamin, a mineral, a botanical extract obtained from plants or cell cultures, an enzyme, a binding agent, a colouring agent, a preservative, a flavour and an aroma.

Preferably the compositions of the invention are for oral administration, more preferably in the form of tablets, pills, hard/soft capsules, solutions, suspensions, emulsions, syrups, granules, elixirs, troches, lozenges, powders (for reconstitution or direct use), effervescent tablets, chewable tablets, sublingual tablets, oral sprays, oral films, and oral gels.

Still more preferably the compositions of the invention, also herein indicated as formulations (F) can be:
- Hesperidin 112 mg/tablet;
- Eriocitrin (from lemon extract) 22 mg/tablet;
- Neoeriocitrin + Neohesperidin (from bergamot extract) as total flavonoids 40 mg/tablet.

In a preferred embodiment the nutraceutical and pharmaceutical compositions for oral administration have a total amount of Hesperidin, Eriocitrin, Neoeriocitrin and Neohesperidin in the range from 348 mg to 522 mg.

These oral formulations are prepared by mixing the flavanones with one or more suitable excipients such as starch, calcium carbonate, sucrose or lactose, gelatin, etc.

In case of liquid formulations for oral administration suspensions, solutions, emulsions and syrups are the preferred embodiments, and various excipients such as wetting agents, sweeteners, aromatics and preservatives in addition to generally used simple diluents such as water and liquid paraffin can be used.

In a still further aspect, the invention relates to a pharmaceutical composition, as herein described, for use as a medicament.

It is also object of the invention the use of a pharmaceutical composition, as herein disclosed, in the prevention and/or treatment of Intestinal Bowel Disease (IBD) and Irritable Bowel Syndrome (IBS).

Preferably, IBD is selected from the group consisting of Ulcerative Colitis or Crohn's Disease and IBS is selected from the group consisting of Celiac Disease and Diverticulitis.

According to another aspect, the described invention provides the use of the nutraceutical composition as a food supplement.

Various embodiments and aspects of the present invention as delineated hereinabove and as claimed in the claims section below find experimental support in the following examples.

### EXPERIMENTAL SECTION

Reference is now made to the following material and methods and examples, which together with the above descriptions illustrate some embodiments of the invention.

### MATERIALS AND METHODS

### In vitro study

### Test solution preparation

The composition according to the present invention (Formulation or F) was prepared on the assumption that *in vitro* simulated digestion (DF), which would then be applied to Caco-2 cell-based models to test the anti-inflammatory and antioxidant activity, had a final concentration of 10 µM, which represents the mean efficacy concentration, considering the half-inhibitory concentration (IC₅₀) values. For this purpose, stock solutions (14 mM) of hesperidin (HED), limon extract (LE) and bergamot extract (BE), solubilized in DMSO, were mixed and diluted 10-fold with Milli-Q water to obtain the 1.4 mM F, which was used to carried out the *in vitro* simulated gastro-intestinal digestion to obtain the 10 µM DF.

### In Vitro Simulated Gastro-Intestinal Digestion

*In vitro* gastric and duodenal digestion of F was carried out. F' solution (1.5 mL), has been dissolved in 7.5 mL of simulated gastric solution containing 0.127 mM egg-phosphatidylcholine and adjusting the pH to 2.5, by 1 M HCI. Gastric pepsin and lipase (9000 U/mL and 60 U/mL, respectively) were added to the mixture starting the gastric digestion phase, which was protracted for 2 h at 37 °C, incubating under continuous stirring (170 rpm) by an Innova 4000 Benchtop Incubator Shaker (New Brunswick Scientific, Edison, NJ, USA). The gastric phase was stopped by the addition of 1M NaOH to reach a pH of 7.5, and 6 mL of the previous solution passed to the duodenal digestion phase. Duodenal solution was prepared by adding 2.10 mL of simulated bile solution (6.5 mM phosphatidylcholine, 4 mM cholesterol, 12.5 mM sodium taurocholate, and 12.5 mM sodium glycodeoxycholate) to 5.9 mL of simulated pancreatic juice containing pancreatic lipase (590 U/mL), colipase (3.2 µg/mL), trypsin (11 U/mL), α-chymotrypsin (24 U/mL), and α-amylase (300 U/mL). The duodenal mixture was incubated for 4 h under continuous stirring at 37 °C as described above. At the end, the DF were immediately stored at -80 °C until the subsequent analyses.

### Inhibitory Activity on Human COX-1 and COX-2

The potential activity of HED, LE, BE and DF in inhibiting human COX enzymes was evaluated by means of the COX (human) Inhibitor Screening Assay Kit (Item No. 701230, Cayman Chemical Company, Ann Arbor, MI, USA). This assay includes human recombinant COX-1 and COX-2, allowing the user to screen specific inhibitors, eliminating the false positive leads generated by less specific methods. The assay was performed according to the manufacturer's recommendations, using hesperidin, LE, BE, DF and nimesulide, as reference selective COX-2 inhibitor, all at the same concentration (10 µM). The results were expressed as COX-1 and COX-2 inhibition percentage (%).

### Cell-based assay

### Cell model

Experiments were carried out on Caco-2 cell monolayers (CacoReady^{™}, Readycell, Barcelona, Spain) according to Denaro et al. 2021. Briefly, 8.5 × 10⁴ cells/cm² Caco-2 cells (passage number 45-55) were seeded on polyester permeable supports (6.5 mm, 0.33 cm², and 0.4 µm) in 24-well HTS plates (Corning Incorporated, Corning, NY, USA). DMEM low glucose (1 g/L) with 10% fetal calf serum (FCS), 2 mM glutamine, 1 U/mL penicillin, and 1 U/mL streptomycin was added onto the apical (0.3 mL) and basolateral side (0.9 mL) of each transwell support. After 21 days of culture incubation at 37 °C, 5% CO₂, and 95% relative humidity, the Caco-2 cells were completely differentiated and polarized, resembling the morphological and functional features of mature enterocytes lining the small intestine. Before conducting the experiments, the monolayer integrity was checked by measuring the trans-epithelial electrical resistance (TEER) with a Millicell^{®} ERS-2 V/ohmmeter (Merck Millipore, Darmstadt, Germany) equipped with an STX 100C electrode (World Precision Instruments, Sarasota, FL, USA). Only Caco-2 monolayers with epithelial resistance ≥ 800 Ω/cm² were used to carried out the experiments.

### Cell treatment

The antioxidant and anti-inflammatory activities of HED, LE and BE, as well as DF were evaluated on a Caco-2 transwell model. In both cases, 25 ng/mL IL-1β was used to trigger oxidation and inflammation events. Co-treatments with HED, LE, BE, DF, nimesulide and trolox as reference standards, all at the same concentration (10 µM for each flavone and reference standard), were carried out on the apical side by diluting the test solutions in completed DMEM (250 µL). Completed DMEM containing 0.1% DMSO and 25 ng/mL IL-1β were used as negative (CTR-) and positive controls (CTR+), respectively. Completed DMEM (0.75 mL) was added on the basolateral side and the cells were incubated for 24 h at 37 °C, 5% CO₂, 95% relative humidity. Cell culture media were collected and stored at -80 °C until the subsequent analyses.

### Detection of PGE2

The activation of the arachidonic acid cascade was evaluated through the production of PGE2 after the addition of the arachidonic acid. Briefly, 24 h after the treatment, the cells were washed thrice with PBS and incubated with 10 mM arachidonic acid in PBS for 10 min. The release of PGE2 into the extracellular medium was quantified (pg/mL) using the Prostaglandin E2 ELISA Kit-Monoclonal (Item No. 514010, Cayman Chemical Company, Ann Arbor, MI, USA) in accordance with the manufacturer's protocol, by using an UV-Vis plate reader (Multiskan GO; Thermo Scientific, MA, USA) set at 405 nm.

### Detection of Oxidative Stress Markers

The determination of the oxidative stress parameters was carried out on cell lysate obtained by pipetting 300 µL of cold 0.1% Triton X-100 on monolayers. The glutathione reduced (GSH) and glutathione disulfide (GSSG) quantification was carried out by solid phase extraction (SPE) followed by HPLC-DAD analysis. Briefly, 400 µL of meta-phosphoric acid was added to 200 µL of cell lysate and incubated at RT for 15 min The sample was then centrifuged at 10,000× g for 5 min at 4 °C, and the supernatant cleaned up using a BOND Elut C18 cartridge 50 mg/3 mL (Agilent Technologies, Santa Clara, CA, USA) pre-conditioned with methanol (2 mL). The elution was carried out with 0.1% TFA (1 mL). The samples were filtered by a 0.22 µm nylon syringe filter and injected into an Agilent 1100 series HPLC system (Santa Clara, CA, USA). The elution was carried out on a Prodigy^{™} 5 µm ODS-3 100 Å LC Column 250 × 4.6 mm (Phenomenex, Torrance, CA, USA) according to Smeriglio et al. 2023. The quali-quantitative analysis was carried out by comparing retention times, UV-Vis spectra (range 190-400 nm), and using the external standard calibration curves (10-10,000 ng/mL) of the commercially available reference compounds (GSH and GSSG) solubilized in mobile phase. The detection of thiobarbituric acid-reactive substances (TBARS) was carried out using the TBARS (TCA Method) assay kit (Item No. 700870, Cayman Chemical, Ann Arbor, MI, USA) following the manufacturer's instructions. This assay was based on the fluorimetric detection of the malondialdehyde (MDA)-thiobarbituric acid (TBA) adduct, which is formed in acidic conditions and high temperatures (90-100 °C), at an excitation (λex) and emission (λem) wavelengths of 530 and 550 nm, respectively, by a fluorescence microplate reader (FLUOstar Omega, BMG LABTECH, Ortenberg, Germany). MDA was used as a reference standard (0.0625-5 µM).

The protein carbonyl content was quantified (nM) by the protein carbonyl colorimetric assay kit (Item No. 10005020, Cayman Chemical, Ann Arbor, MI, USA) based on the 2,4-dinitrophenylhydrazine reaction, according to the manufacturer's instructions. The amount of protein-hydrazone produced was quantified spectrophotometrically at 370 nm.

The ROS levels, expressed as percentage (%), were assessed by measuring the fluorescence resulting from the intracellular oxidation of DCF-DA (10 µM in PBS), which was added to the culture medium 30 min before ending the cell treatment. The medium was then removed, and the cells washed five times with PBS (pH 6.7). The cell lysates were diluted with PBS and the fluorescence recorded at λₑₓ 485 and λₑₘ 535. All data were normalized for protein concentration detected using the protein determination (BCA) kit (Item No. 701780, Cayman Chemical, Ann Arbor, MI, USA) according to the manufacturer's instructions.

### Post-quality control assays

TEER measurement, as well as the apparent permeability coefficient (Pₐₚₚ) and paracellular flux (P_{f}) of lucifer Yyellow (LY) detection, were assessed to evaluate the Caco-2 monolayer integrity. Cell viability was assessed by MTT assay.

### Evaluation of the Synergistic Effect by CompuSyn Software

Because the F was prepared by combining HED, LE and BE (7:1:2), the potential synergistic effect of DF in all the antioxidant and anti-inflammatory assays carried out in the present study was investigated applying the method of the constant ratio. For this purpose, five different concentrations of DF (2.5, 5, 7.5, 10, and 20 µM) were tested, maintaining the constant ratio at 7:1:2 (0.875-14 µM for HED, 0.125-2 µM for LE, and 0.25-4 µM for BE) to simulate the hypotized formulation. In addition, the same experiments with HED, LE and BE at the same concentrations of DF (2.5, 5, 7.5, 10, and 20 µM), were run as a control. The activity values obtained for all assays, expressed as percentages, and converted into the fraction of effect (Fa) according to the following equation: Fa = 100% of activity/100, were used for the calculation of the synergism using CompuSyn software Version 1.0 (ComboSyn, Inc., Paramus, NJ, USA). The data points, including treatment concentrations (µM) and Fa, were automatically processed. The results, expressed as combination index (CI), quantitatively determine whether a synergism (Cl < 1), an additive effect (Cl = 1), or an antagonism (Cl > 1) occurs in the specific experimental conditions.

### In vivo study

### Experimental model

Ninety 7-week-old male C57BL/6 mice, weighing 22-25 g, were housed under a 12-h light/dark cycle for 7 days before starting the experiment, and then randomized into 6 groups (*n* = 15, Table 1).

**Table 1. Treatment plan**

| **Groups** | | **Mice n.** |
|---|---|---|
| I. | Sham: deionized water (CTR-) | 15 |
| II. | Sham + vehicle: 30% PEG 400 (CTR+) | 15 |
| III. | Reference drug: 100 mg/kg Sulfasalazin (SULF) | 15 |
| IV. | Treatment I: 100 mg/kg formulation (F) | 15 |
| V. | Treatment II: 50 mg/kg formulation (F) | 15 |
| VI. | Treatment III: 25 mg/kg formulation (F) | 15 |

Ulcerative colitis (UC) was induced in all treated groups by *ad libitum* administration of 3% (w/v) dextran sulfate sodium (DSS) dissolved in deionized water for 7 days, except for the Sham which received only deionized water. From the 3^{rd} day to each group was administered, *via gavage,* every 24 hours for the following 7 days, its treatment at the above-mentioned dose, as schematically represented in Figure 1. On the 10^{rd} day, the mice were fasted, anesthetized and sacrificed with chloral hydrate. Immediately after sacrifice, the entire colon (from the cecum to the rectum) was recovered, measured and weighed after being opened longitudinally and washed with phosphate buffer (10 mM PBS, pH 7). Colon samples were stored partially in formalin for the histological analyses, and partially frozen at -80 °C until further analyses.

The *in vivo* study, approved by the ethical committee (D01N01UN280120200002), was carried out according to Directive 2010/63/EU of the European Parliament and of the Council of 22 September 2010 on the protection of animals used for scientific purposes and in accordance with the ARRIVE guidelines 2.0. The minimum number of animals for each group (n = 15) was calculated with the G*Power software version 3.1.9.4 (Heinrich-Heine-Universität Düsseldorf), according to the parameters reported in Figure 2. The mice were randomized with the "simple randomization" technique.

### Assessment of colon damage and calculation of disease activity index (DAI)

During the study (10 days), the following parameters were measured and evaluated daily: body weight, stool consistency and faecal bleeding. All information was collected in a clinical evaluation form specifically prepared for each animal (Figure 3) and the disease activity index (DAI) calculated according to the scores reported in Table 2.

**Table 2. Parameters for the calculation of the DAI and related scores.**

| **Punteggio** | **Weight loss (%)** | **Stool consistency** | **Faecal bleading** |
|---|---|---|---|
| 0 | Absent | Normal | Absent |
| 1 | 1-5 | Wet/Sticky | Minimum |
| 2 | 5-10 | Soft | Medium |
| 3 | 10-20 | Very soft | Prominent |
| 4 | >20 | Diarrhea | Diffuse bleeding |

### Histological analyses

### Sample preparation

Colon samples were fixed in buffered formalin immediately after collection and left for 48 hrs. Subsequently, they were cut to obtain two transverse segments of colonic wall (along the major colon axis) with a length between 1 and 2 cm for each animal. The samples were then treated with a LOGOS hybrid instrument (Milestone) that performs the complete sample processing cycle (fixation, dehydration, clearing and paraffinization) in 13 h and 56'. The processed samples were manually embedded in liquid paraffin and then cut into microtomic sections of 3-4 microns thick with a LEICA RM2255 microtome. For each inclusion, three microtomic sections were cut, placed on Superfrost slides (Thermo scientifc), and stained with Hematoxylin and Eosin using a Ventana He600 automatic stainer (Roche Diagnostic). The stained slides were analyzed with a microscope (Olympus Bx51) equipped with a digital camera (Olympus DP70).

### Sample evaluation

The samples were blinded evaluated by two pathologists, expert in gastrointestinal disorders and murine models.

The following histological variables were analyzed:
A) Transverse extension (mm) of ulcerative mucosal lesions with calculation of the relative percentage with respect to the whole sample;
B) Severity of the lesions using the scoring system.

This scoring system specifically evaluates:
1) Degree of inflammation (0, absent; 1, mild; 2, moderate; 3, severe);
2) Extension of damage (0, absent; 1, damage limited to the mucosa; 2, damage extended to the submucosa; 3, damage extended to the muscularis propria and serosa);
3) Damage to the crypts (0, no damage; 1, loss of 1/3 of the crypts; 2, loss of 2/3 of the crypts; 3, loss of 100% of the crypts; 4, loss of 100% of the crypts and epithelium).
4) Extent of lesions (0, no lesions; 1, extension of lesions from 1 to 25% of the surface; 2, extension of lesions from 26 to 50% of the surface; 3, extension of lesions from 51 to 75% of the surface; 4, extension of lesions from 76 to 100% of the surface).

### Pro-oxidant and pro-inflammatory marker analyses

### Sample preparation

Colon samples were weighed and homogenized in an ice bath with 2 mL of 10 mM PBS. The obtained homogenates were sonicated using a titanium probe (3 mm), set to 200 W and 30% amplitude (Vibra Cell^{™} Sonics Materials, inc., Danbury, Connecticut, U.S.A.) for 5 min in an ice bath, and centrifuged at 10,000 *x* g for 30 min at 4 °C. The supernatants were then collected and stored at -80 °C until analysis.

### Total protein determination

The sample total protein content was determined by Bradford reagent. Briefly, in a 96-well plate, 5 µL of sample were added to 150 µL of Bradford reagent, and after 5 min, the absorbance was recorded at 595 nm by a spectrophotometric plate reader (Multiskan Go, Thermo Scientific, Waltham, MA, USA). The results were expressed as mg of protein/mg of colon, by using bovine serum albumin as standard.

### Evaluation of the pro-inflammatory markers

Myeloperoxidase (MPO) (Biovision K744-100, BioVision Incorporated, 155 S. Milpitas Boulevard, Milpitas, CA 95035 USA), malondialdehyde (MDA) (Cayman Chemical, 700870, 1180 E. Ellsworth Rd. Ann Arbor, MI, USA) and carbonyl groups (Cayman Chemical, 10005020, 1180 E. Ellsworth Rd. Ann Arbor, MI, USA) were evaluated according to the manufacturer's instructions. Results were expressed as mU MPO/mg protein, µM MDA/mg protein and nmol carbonyl groups/mg of protein, respectively. Nitric oxide (NO) release was assessed according to Smeriglio et al. 2023. Briefly, 100 µL of sample were added to 100 µL of Griess reagent (1% sulfanilamide (*w*/*v*) in 5% phosphoric acid (*v*/*v*) and 0.1% naphthylethylenediamine-HCl (1:1 *vlv*) and incubated for 10 min at RT. Absorbance was recorded at 550 nm using a plate reader (Multiskan GO, Thermo Scientific, Waltham, MA, USA). NO was quantified using sodium nitrite as reference standard (1.0 - 15 µM). Results were expressed as µmol NO/mg protein.

### Evaluation of the pro-inflammatory markers

Tumour necrosis factor-α (TNF-α) (Cayman Chemical, 589201, 1180 E. Ellsworth Rd. Ann Arbor, MI, USA), interleukin (IL)-1β (Cayman Chemical, 583311, 1180 E. Ellsworth Rd. Ann Arbor, MI, USA) and IL-6 (Cayman chemical, 5010301180 E. Ellsworth Rd. Ann Arbor, MI, USA) were analysed according to the manufacturer's instructions. Results were expressed as pg TNF-α/mg protein, pg IL-1β/mg of protein and pg IL-6/mg of protein, respectively.

### Western blotting analysis

Colons were homogenized on ice with lysis buffer (150 mM NaCl, 20 mM Tris pH 7.5, 1 mM EDTA, 0.5% sodium deoxycholate, 0.1% SDS, and 1% nonidet P-40) containing a mix of proteinase (Roche) and phosphatase inhibitors (Sigma). Samples were then centrifuged at 12,500 rpm for 30 min at4°C, and the supernatant was collected and stored at -80°C until further analysis. Protein concentration was determined by the Bradford assay (Bio-Rad, Hercules, CA) using bovine serum albumin as a standard. Equal amounts of protein for each sample (20 mg) were electrophoresed on 8% and 10% Tris-glycine gels using a 4% stacking gel. The separated proteins were transferred onto polyvinylidene fluoride (PVDF) membranes blocked with 5% bovine serum albumin for 1 h. IL-1β, IL-6 and TNF-α (diluted 1:1000, Cell Signaling Technology, Danvers, MA) were used as primary antibodies. Horseradish peroxidase conjugated to anti-rabbit IgG was used as secondary antibody (diluted 1:7000 in blocking solution). An enhanced chemiluminescence kit consisting of horseradish peroxidase, enhanced luminol, and oxidizing reagents (Bio-Rad) was used to identify protein expression. Bands were photographed in the darkroom using a CCD camera (Molecular Imager Gel Doc XR System; Bio-Rad). The thickness of the same (or intensity of the area) was quantified using the Quantity One software (Bio-Rad).

### Statistical analysis

For each test, three independent experiments were performed in triplicate (*n*=3). The results, expressed as mean ± standard deviation (SD), were analyzed by one-way analysis of variance (ANOVA) followed by Tukey and Student-Newman-Keuls test using SigmaPlot12 software (Systat Software, Inc., San Jose, CA, USA). The results were considered statistically significant at *p* ≤ 0.05.

### EXAMPLES

### Example 1. In vitro cell-free and cell-based assays

### In Vitro Inhibitory Activity on Human COX-1 and COX-2

The enzymatic assay carried out on human COX-1 and COX-2 highlighted different behaviours between HED, LE, BE and DF (Table 3).

**Table 3. Inhibitory activity of 10 µM hesperidin (HED), Lemon Extract (LE, 10 µM eriocitrin), Bergamot Extract (BE, 10 µM neoeriocitrin and 10 µM neohesperidin), 10 µM digested formulation (DF, 7:1:2 for HED, LE and BE), and 10 µM nimesulide (NIM), as reference standard, on human COX-1 and COX-2 enzyme' isoforms.**

| **Sample** | **Inhibitory activity (%)** | |
|---|---|---|
| | COX-1 | COX-2 |
| HED | 53.30 ± 0.17 ^{abc} | 55.10 ± 0.20 ^{ab} |
| LE | 3.14 ± 0.02 ^{abc} | 57.55 ± 0.51 ^{ab} |
| BE | 22.71 ± 0.32 ^{abc} | 75.59 ± 0.26 ^{ab} |
| DF | 46.23 ± 0.50 ^{ac} | 83.43 ± 0.10 ^{a} |
| NIM | 0.31 ± 0.03^{bc} | 88.15 ± 0.16^{b} |

| | | |
|---|---|---|
| ^{a}*p*<0.001 vs. NIM; ^{b}*p*<0.001 vs. DF; ^{c}*p*<0.001 vs. COX-2 | | |

Indeed, HED showed a similar trend, being active on both COX isoforms, with an inhibition of about 50%. On the contrary, LE and BE showed a higher inhibitory activity on COX-2 (57.55 % and 75.59 %, respectively), with BE which showed the highest selectivity towards COX-2. All treatments showed statistically significant results (p < 0.001) with respect to nimesulide, used as a reference standard, which showed a marked selectivity for COX-2. Interestingly, by analysing the data reported in Table 3, it can be noted that DF, while maintaining the activity on COX-1, shown mainly by HED, it showed a greater selectivity for COX-2, and was even higher in terms of inhibition percentage with respect to HED, LE and BE. This demonstrates, once more, how the 10 µM formulation exhibits increased activity with respect to tested HED, LE, BE, at the same concentration. Finally, it is interesting to note how the activity on COX-2 is always significantly higher (p < 0.001) for all investigated treatments, in particular for BE (10 µM neoeriocitrin and 10 µM neohesperidin).

### Anti-Inflammatory Activity

Given the marked inhibitory activity found *in vitro* in the preliminary enzymatic tests on human COX isoforms, the ability of HED, LE, BE and DF to inhibit prostaglandin release after treatment with IL-1β and arachidonic acid was evaluated on a Caco-2 cell-based model. Nimesulide (10 µM) was used, once again, as a reference standard. The results, reported in Figure 4, showed a significantly (p < 0.001 vs. CTR-) increase of PGE2 release following treatment with IL-1β (CTR+).

All treatments significantly decreased PGE2 release with respect to CTR+. Interestingly HED, BE and LE, as well as DF (p < 0.05) showed a significantly decrease with respect to 10 µM nimesulide. Furthermore, it seems that among the single treatments, BE (10 µM neoeriocitrin and 10 µM neohesperidin) is, once again, the main responsible for this activity.

### Antioxidant Activity

An imbalance of the redox homeostasis with a ROS overproduction is often associated with the inflammatory phenomenon. The ability of HED, LE, BE and DF to maintain this subtle balance by hindering the pro-inflammatory process induced by IL-1β, was evaluated on the same cellular model by recording the changes in four key oxidative stress markers: carbonylated proteins, TBARS, ROS release, and GSH/GSSG ratio. Trolox was used as reference standard due to its proven antioxidant activity. As shown in Figure 5, IL-1β caused a significant increase in the protein carbonyl content, TBARS and ROS release, and a significant decrease in the GSH/GSSG ratio with respect to CTR-. DF showed the strongest antioxidant activity in all four tests performed, showing, at the same concentration (10 µM), an activity comparable to the trolox in terms of TBARS, or even higher (*p* < 0.05 vs TRX) in the case of carbonylated proteins, ROS release and GSH/GSSG ratio.

According to previous results, a combination effect is evident. Indeed, if tested alone at the same concentration (10 µM), HED, LE and BE always show a significantly lower antioxidant activity (*p* < 0.05) than DF. Interestingly, according to the first mentioned results on PGE2 release, BE seems to play a pivotal role in the DF activity (Figure 5).

### Post-quality control assays

No cytotoxicity (cell viability ≥ 98.27% ± 2.58) or alteration of the cell membrane permeability (TEER ≥ 800 W/cm², LY Pₐₚₚ ≤ 1.08 × 10⁻⁶ cm/s and P_{f} ≤ 0.42%) were detected in the Caco-2 monolayers after the antioxidant and anti-inflammatory assays, with recorded values remaining within the reference standard range.

### Evaluation of the Synergistic Effects

The potential synergistic effect of DF was investigated using constant ratio experiments. For this purpose, all the antioxidant and anti-inflammatory assays carried out on the target concentration (10 µM) were repeated with three lower concentrations (2.5, 5 and 7.5 µM) and one higher concentration (20 µM), to follow the DF behaviour at five concentrations (Figure 6).

The synergistic effect of DF (7:1:2 as µM for HED, LE and BE) was analyzed by CompuSyn software, which generates computerized simulation data from various concentrations and fractions of effect (Fa). The simulated data were simplified as a combination index plot (Cl-Fa, Figure 6A-G). The synergistic effect of DF was detected at every concentration point tested in each experimental condition (Figure 6A-G). Furthermore, it is interesting to note that, the antioxidant and anti-inflammatory activity of DF remained unchanged within the tested concentration range (2.5 to 20 µM) (Figure 6). The results indicate that, at this constant ratio (7:1:2 as µM for HED, LE and BE), DF showed synergy on a broad range of effects showing IC values ≤ 0.90 with a confident limit of 90% for all tested experimental conditions.

### Example 2. In vivo study: evaluation of the intestinal anti-inflammatory activity of planned formulation

### Disease activity index (DAI)

Mice exposed to oral administration of 3% DSS for 7 days were characterized by sustained weight loss (Figure 7A), abnormal stool consistency (Figure 7B), and bloody diarrhoea (Figure 7C), which led to a significant increase of DAI score, considered a key indicator of the success of the used UC model (Figure 7D).

To evaluate the preventive and therapeutic effects of formulation (F) and to compare it with the reference drug, mice were treated during the induction and development of UC with three different doses of F (25, 50, and 100 mg/kg/day) and with 100 mg/kg/day sulfasalazine (SULF). All mice groups treated with F, as well as the SULF group, showed significantly (*p* < 0.05) decrease of the DAI score, compared to UC control group (CTR+) (Figure 7D).

Furthermore, since colon length is inversely correlated with UC severity and it is considered an indirect inflammation marker, the colonic shortening was also examined. Again, all three F tested doses (25, 50 and 100 mg/kg/day) as well as sulfasalazine (SULF 100 mg/kg/day), showed a significantly beneficial effect on DSS-induced colon shortening (Figures 7E and 7F). Furthermore, it is interesting to note that sulfasalazine treatment (SULF 100 mg/kg/day) showed no statistically significant results with respect to the F doses 50 and 100 mg/kg/day, in all parameters used for the calculation of the DAI score (Figure 7D), except for weight loss (Figure 7A).

### Histological analyses

The results of the histological evaluation carried out on the colons of treated (CTR+; SULF 100 mg/Kg/day; F 100, 50 and 25 mg/Kg/day) and untreated (CTR-) mice according to the reference scoring system, were shown in Table 4.

**Table 4. Score evaluation of the colon histological features of the treated (3% DSS, CTR+; SULF 100 mg/Kg/day; F 100, 50 and 25 mg/Kg/day) and untreated (vehicle, CTR-) mice.**

| **Group** | **Lesion extent** mm and % | **Inflammation** 0-3 | **Damage extent** 0-3 | **Crypt damage** 0-4 | **Lesion range** 0-100 |
|---|---|---|---|---|---|
| CTR + | 8/21 mm, 38.09% | 3 | 3 | 4 | 26-50 |
| SULF | 9/26 mm, 34.61% | 3 | 2 | 3 | 1-25 |
| F 100 | 1.5/29 mm, 5.17% | 1 | 1 | 2 | 1-25 |
| F 50 | 2/22 mm, 9.09% | 3 | 2 | 2 | 1-25 |
| F 25 | 6/21 mm, 28.57% | 3 | 3 | 3 | 1-25 |
| CTR- | 0/30 mm, 0% | 0 | 0 | 0 | 0 |

Analysing the obtained results (Table 4), it is immediately evident that F at the dose of 50 mg/Kg/day is the one most comparable to the treatment with SULF 100 mg/Kg/day. The highest dose of the formulation determines a substantial decrease of all the investigated parameters, whereas the lowest dose appears more comparable to CTR+, but with a lower loss of crypts. What appears noteworthy is that the range of lesion appears substantially reduced with any dose of the formulation used (Table 4). According to these results, in untreated control mice (CTR-, Figure 8A panel a and b), no significant inflammatory lesions were observed, and the colonic mucosa' morphology was normal with the presence of numerous goblet cells. On the contrary, 3% DSS-treated mice (CTR+, Figure 8B panel a and b) showed extensive mucosal ulcerations with transmucosal extension, up to and including the *muscularis propria,* with circumferential extension (black arrows). The inflammation was characterized by mono- and polymorphonuclear elements with granulation tissue, loss of crypts and of lining epithelium (grey arrow).

In sulfasalazine-treated mice (Figure 8C panel a and b), less extensive ulcerations were observed, with transmucosal extension, involvement of the *submucosa* and, sometimes circumferential (black arrows). Nevertheless, the lining epithelium was partially preserved, and the inflammation consisted of mono- and polymorphonuclear elements with granulation tissue and loss of crypts (grey arrow). In mice treated with the F highest dose (F 100 mg/kg, Figure 8D panel a and b), a focal ulceration with trans-parietal extension, but very limited longitudinally, was observed (black arrow). Marked reactive regenerative aspects of the surrounding epithelium, with complete re-epithelialization of some ulcers, were observed (white arrows). The lining epithelium appeared preserved, whereas the inflammatory infiltrate, consisted of mono- and polymorphonuclear elements with granulation tissue and moderate loss of crypts, were significantly decreased with respect to CTR+ (grey arrow).

In F 50 mg/kg-treated mice (Figure 8E panel a and b), less extensive ulcerations still with transmucosal extension, with involvement of the submucosa but without circumferential extension, were observed (black arrow). The inflammatory infiltrate, consisted of mono- and polymorphonuclear elements with granulation tissue and loss of crypts, appeared more extensive (grey arrow). A focal reactive regenerative aspect was recorded (white arrow).

Finally, in mice treated with the lowest dose of the formulation (F 25 mg/kg, Figure 8F panel a and b) there was a focal ulceration with transmucosal extension, without any clear evidence of extension to the submucosa, and very limited longitudinally (black arrow). Although inflammatory infiltrates, consisted of mono- and polymorphonuclear elements with granulation tissue and loss of crypts, were present within the *muscularis mucosae* (grey arrow), marked reactive regenerative aspects of the surrounding epithelium were observed (white arrow).

### Pro-oxidant and pro-inflammatory markers analysis

To better understand how F was able to reduce intestinal inflammation *in vivo,* the release of different pro-oxidative and pro-inflammatory markers from colon samples was analysed by immuno-enzymatic and colorimetric assays (Figure 9 and 10, respectively).

As depicted in Figure 9, 3% DSS (CTR+) significantly increased (*p* < 0.05) the MDA, carbonyl groups, MPO and NO levels with respect to CTR-. SULF 100 mg/Kg/day, as well as all F doses (25, 50 and 100 mg/Kg/day), significantly decreased (*p* < 0.05) the investigated pro-oxidant marker levels (Figure 9). Interestingly, the highest dose of F (100 mg/kg/day) showed a significantly decrease (*p* < 0.05) in MDA, carbonyl groups and NO levels, even compared to sulfasalazine (SULF 100 mg/Kg/day), and comparable values for MPO. On the contrary, FM 50 mg/Kg/day, showed a significantly decrease with respect to SULF 100 mg/Kg/day only for carbonyl groups levels. However, what is interesting to note, is that, in all investigated parameters, a dose-dependent and statistically significant (*p* < 0.05) F behaviour, was recorded. According to previous results, the analysis of the pro-inflammatory markers showed that treatment with 3% DSS (CTR+) significantly increased (*p* <0.05) the TNF-α, IL-1β and IL-6 release with respect to the negative control (CTR-) (Figure 10). Sulfasalazine (SULF 100 mg/Kg/day) significantly decrease (*p* < 0.05) these events, with values ranging from 2 to 26 times lower with respect to CTR+, with the following order of efficacy: TNF-α, IL-6 and IL-1β (Figure 10).

Similarly, even the F highest dose (100 mg/Kg/day) significantly (*p* < 0.05) decrease all investigated pro-inflammatory markers release with values ranging from 9 to 25 times lower with respect to CTR+ (Figure 10). Furthermore, it showed a significantly behaviour (*p* < 0.05) with respect to the reference drug sulfasalazine (SULF 100 mg/Kg/day) in all the investigated parameters, except for MPO.

The order of efficacy, however, is different if compared to sulfasalazine: IL-1β, TNF-α and IL-6, leading to postulate the F pivotal role to target IL-1β, which is well-known to possess a crucial role in the promotion of inflammation and immune responses in IBD. This trend was also observed at F lowest tested doses (50 and 25 mg/Kg/day), with a dose-dependent behaviour like that observed with the pro-oxidative stress markers (Figure 10).

These last results were corroborated also by western blotting analyses as shown in Figure 11.

## Claims

1. A nutraceutical composition consisting of:
- Hesperidin;
- Eriocitrin;
- Neoeriocitrin
- Neohesperidin; and
- at least one physiologically acceptable excipient,
wherein said Eriocitrin, Neoeriocitrin and Neohesperidin are from a *Citrus* extract.

2. A pharmaceutical composition consisting of:
- Hesperidin;
- Eriocitrin;
- Neoeriocitrin
- Neohesperidin; and
- at least one physiologically acceptable excipient,
wherein said Eriocitrin, Neoeriocitrin and Neohesperidin are from a *Citrus* extract.

3. The nutraceutical composition according to claim 1 or the pharmaceutical composition according to claim 2, wherein said *Citrus* extract is selected from the group consisting of lemon extract or bergamot extract.

4. The nutraceutical composition according to claims 1 or 3, or the pharmaceutical composition according to claims 2-3, wherein
- said Eriocitrin is from lemon extract; and
- said Neoeriocitrin and Neohesperidin are from bergamot extract.

5. The nutraceutical composition according to any one of claims 1, 3-4, or the pharmaceutical composition according to any one of claims 2-4, wherein
- said Hesperidin is in the range from 50 mg to 300 mg;
- said Eriocitrin is in the range from 10 mg to 60 mg; and
- said Neoeriocitrin and said Neohesperidin are in the range from 20 mg to 100 mg.

6. The nutraceutical composition according to any one of claims 1, 3-5, or the pharmaceutical composition according to any one of claims 2-5, wherein
- said Hesperidin is in the amount of 112 mg;
- said Eriocitrin is in the amount of 22 mg; and
- said Neoeriocitrin and Neohesperidin is in the total amount of 40 mg.

7. The nutraceutical composition according to any one of claims 1, 3-6, or the pharmaceutical composition according to any one of claims 2-6, wherein said physiologically acceptable excipient is selected from the group consisting of an auxiliary agent, a filler, an amino acid, a protein, a fatty acid, a carbohydrate, a vitamin, a mineral, a botanical extract obtained from plants or cell cultures, an enzyme, a binding agent, a colouring agent, a preservative, a flavour and an aroma.

8. The nutraceutical composition according to any one of claims 1, 3-7, or the pharmaceutical composition according to any one of claims 2-7, wherein said compositions are for oral administration, preferably in the form of tablets, pills, hard/soft capsules, solutions, suspensions, emulsions, syrups, granules, elixirs, troches, lozenges, powders (for reconstitution or direct use), effervescent tablets, chewable tablets, sublingual tablets, oral sprays, oral films, and oral gels.

9. The nutraceutical composition according to any one of claims 1, 3-8, or the pharmaceutical composition according to any one of claims 2-8, wherein said compositions for oral administration have a total amount of Hesperidin, Eriocitrin, Neoeriocitrin and Neohesperidin in the range from 348 mg to 522 mg.

10. A pharmaceutical composition according to any one of claims 2-9, for use as a medicament.

11. A pharmaceutical composition according to any one of claims 2-9, for use in the prevention and/or treatment of Intestinal Bowel Disease (IBD) and Irritable Bowel Syndrome (IBS).

12. The pharmaceutical composition according to claim 11, wherein said IBD is selected from the group consisting of Ulcerative Colitis or Crohn's Disease.

13. The pharmaceutical composition according to claim 11, wherein said IBS is selected from the group consisting of Celiac Disease and Diverticulitis.

14. Use of the nutraceutical composition according to any one of claims 1, 3-9 as a food supplement.
